# EUROPEAN PATENT SPECIFICATION

(11) **EP 3 442 995 B1**
(45) Date of publication and mention of the grant of the patent: **11.09.2019**
(21) Application number: 17709039.6
(22) Date of filing: 03.03.2017
(51) Int. Cl.: C07K 14/415, C07K 14/47

(54) **PRODUCTION OF SUBERIN**
HERSTELLUNG VON SUBERIN
PRODUCTION DE SUBÉRINE

(30) Priority: 14.04.2016 LU 93026
(43) Date of publication of application: 20.02.2019
(73) Proprietor: Luxembourg Institute of Science and Technology (LIST), 4362 Esch-sur-Alzette (LU)
(72) Inventor: LEGAY, Sylvain, 57000 Metz (FR)
(74) Representative: Lecomte & Partners
(86) International application number: PCT/EP2017/055096
(87) International publication number: WO 2017/178150

(56) References cited:
- LEGAY SYLVAIN ET AL: "Apple russeting as seen through the RNA-seq lens: strong alterations in the exocarp cell wall", PLANT MOLECULAR BIOLOGY, SPRINGER, DORDRECHT, NL, vol. 88, no. 1, 19 March 2015 (2015-03-19) , pages 21-40, XP035492751, ISSN: 0167-4412, DOI: 10.1007/S11103-015-0303-4 [retrieved on 2015-03-19]
- DYLAN K. KOSMA ET AL: "AtMYB41 activates ectopic suberin synthesis and assembly in multiple plant species and cell types", THE PLANT JOURNAL, vol. 80, no. 2, 26 August 2014 (2014-08-26), pages 216-229, XP055324163, GB ISSN: 0960-7412, DOI: 10.1111/tpj.12624
- DANIEL J. GIBBS ET AL: "At MYB93 is a novel negative regulator of lateral root development in Arabidopsis", NEW PHYTOLOGIST, vol. 203, no. 4, 1 September 2014 (2014-09-01), pages 1194-1207, XP055325499, GB ISSN: 0028-646X, DOI: 10.1111/nph.12879
- U LOKESH ET AL: "Transcriptional regulation of functional genes involved in cuticular wax biosynthesis by MYB family transcriptional factors in plants", INTERNATIONAL JOURNAL OF ENVIRONMENTAL & AGRICULTURE RESEARCH, vol. 2, no. 3, 1 March 2016 (2016-03-01), pages 86-92, XP055326257, ISSN: 2454-1850
- SOLLAPURA J. VISHWANATH ET AL: "Suberin: biosynthesis, regulation, and polymer assembly of a protective extracellular barrier", PLANT CELL REPORTS, vol. 34, no. 4, 14 December 2014 (2014-12-14), pages 573-586, XP055325516, DE ISSN: 0721-7714, DOI: 10.1007/s00299-014-1727-z

## Description

### Technical field

The invention is directed to the production of suberin, the depolymerisation process of suberin and to the use of a transcription factor as activator for the production of suberin.

### Background art

Suberin is a natural aliphatic-aromatic cross-linked polyester, almost ubiquitous in the vegetable realm, albeit in very variable proportions. It is mostly found in the cell walls of normal and wounded external tissues of aerial and/or subterranean parts of plants where it plays the fundamental role of a protective barrier between the organism and its environment (see studies of Gandini A. et al, Prog. Polym. Sci., 2006, 31, 878-892).

The main components of the aliphatic domains of suberin are long chains of ω-hydroxyfatty acids, α-, ω-dicarboxylic acids and homologous mid-chain di-hydroxy or epoxy derivatives, whereas the aromatic domains are dominated by variously substituted phenolic moieties.

Suberin acts as a highly efficient barrier which limits water, solute, gas and ion exchange. Indeed, suberized cell walls could act as barriers to minimize the movement of water and nutrients, thus restricting pathogen invasion and impeding toxic gas diffusion. In addition, suberized cell walls provide a barrier to radial oxygen loss from roots to the anaerobic root substrate in wetland plants (see studies of Ranathunge K. et al., Plant Science, 2011, 180, 399-413).

Suberin is particularly abundant in the oak cork, birch outer barks and potatoes. However, these resources are natural, and therefore limited.

The research of Legay S. *et al.* has shown that, while a strong correlation between the expression of a MYB93 transcription factor and key suberin biosynthetic genes such as GAPT5, CYP86A1 and CYP86B1 exists, the function of said MYB93 transcription factor is not known (Legay S. et al., Plant Mol. Biol., 2015, 88, 21-40).

The main utility of suberin and/or of its monomers is the fabrication of polyurethane and/or tackifiers, *i.e.* chemical compounds used in formulating adhesives to increase the stickiness of the surface of the adhesive. These tackifiers are mainly used in the tyre industry.

### Summary of invention

### Technical Problem

The invention has for technical problem to provide an industrial source of suberin and suberin monomers which supplements the limited natural resources.

### Technical solution

A first object of the invention is in accordance with independent claim 1. Further developments are in accordance with dependent claims 2 and 3.

A first example is directed to a process for the production of a suberin polymer, comprising the steps of (a) introducing an orthologous gene of MYB93 transcription factor into plant cells from a plant organism and/or a plant tissue leading to genetically engineered plant cells; (b) stimulating said genetically engineered plant cells; and, (c) isolating a suberin polymer that is produced as result of step (b).

According to an example, said step (a) is performed by *Agrobacterium-*mediated method, by viral-mediated method, by gene gun process or by electroporation, preferably by *Agrobacterium*-mediated method.

According to an example, said *Agrobacterium-*mediated method is performed by inserting at least one vector comprising an orthologous gene of MYB93 transcription factor into a bacterium and by infecting said plant cells from a plant organism and/or a plant tissue with said bacterium, wherein said bacterium is *Agrobacterium tumefaciens.*

According to an example, said at least one vector further comprises a constitutive promotor or an inducible promotor.

According to an example, said constitutive promotor is CaMV 35S.

According to a preferred embodiment, said inducible promotor is induced by oestrogens, ethanol or by a heat shock.

According to a preferred embodiment, said step (b) is performed with hormones, preferably oestrogens.

The second object of the invention is directed to a process for the production of monomers of suberin, comprising (a) the process of the production of suberin in accordance with the first object of the invention and the subsequent steps of (b) removing the lipids contained in the suberin so as to produce a delipidized suberin; and (c) depolymerizing said delipidized suberin so as to produce the monomers of suberin.

According to a preferred embodiment, said step (c) is performed by ester-breaking chemical reactions.

According to a preferred embodiment, said plant organisms are tobacco plant and/or apple plant, and/or wherein said plant tissues are leaves, fruits, roots and/or stems.

According to an example, the coding sequence of orthologous gene of MYB93 transcription factor is the genes MDP0000320772 (SEQ-ID NO:1) and/or MDP0000228252 (SEQ-ID NO:2).

A third object of the invention is in accordance with independent claim 7.

An example concerns the use of orthologous gene of MYB93 transcription factor as activator for regulating with an inducible promotor the expression of SMT1, KCS1, KCS4, 4CL1, 4CL3, AT2G39350, WBC11, AT3G55090, AT2G37360, AT5G19410, PDR4, AGL21, AT4G24140, AT4G36610, AT4G27450, ANNAT8, AT2G03200, AT4G32480, AT4G17800, CT-BMY, BETA-TIP, AT2G44300, AT3G22620, GPAT4, GPAT6, BT4, CSLC12, AT5G38200, AT4G15610, AT5G44550, AT5G07475, CYP94C1, CYP96A10, CYP86A8, CYP86A1, CYP86B1, AT5G11880, IRX12, ENODL17, AT2G18360, WRI1, CER4, DIN10, AT1G74460, AT5G03610, AT5G22810, AT5G37690, AT1G75900, AT2G23540, AT5G23370, ASN1, GPAT5, AT5G49350, GH9C1, AT1G80160, AT5G24080, AT4G27300, Hsp70b, AT5G24580, AT1G29000, AT1G71050, HHP1, AT1G32910, AT5G09520, LAC12, AT5G48480, AT1G54540, AT3G22800, AT5G13900, LACS4, TAA1, AT2G23790, SKS1, AT4G15700, MYB67, MYB93, MIOX2, NAC038, NAC058, NAC073, NAC075, AT1G09380, LP1, LTP4, NAT7, AT3G49190, OLEO3, AT4G35160, AT3G59850, PE11, AT5G19640, AT3G52790, AT1G68850, AT5G05340, AT5G14130, PAL2, AT3G09925, AT5G47635, IRX9, XTR6, AT3G22600, AT1G05450, BST1, CER3, EDA4, EXO, OXS3, AT5G09530, RPD1, TMN7, TBL19, TBL33, AT2G14830, AT5G23750, AT5G41400, ATL8, RIC4, AT2G33205, AT5G18840, SULTR1, AT5G11620, MYB84, UCC3, PGSIP3, AT5G27020, AT4G10265 and/or AT1G68360 gene in plant organisms and/or in plant tissues.

According to a preferred embodiment, the expression of SMT1, KCS1, KCS4, 4CL1, 4CL3, AT2G39350, WBC11, AT3G55090, AT2G37360, AT5G19410, PDR4, AGL21, AT4G24140, AT4G36610, AT4G27450, ANNAT8, AT2G03200, AT4G32480, AT4G17800, CT-BMY, BETA-TIP, AT2G44300, AT3G22620, GPAT4, GPAT6, BT4, CSLC12, AT5G38200, AT4G15610, AT5G44550, AT5G07475, CYP94C1, CYP96A10, CYP86A8, CYP86A1, CYP86B1, AT5G11880, IRX12, ENODL17, AT2G18360, WRI1, CER4, DIN10, AT1G74460, AT5G03610, AT5G22810, AT5G37690, AT1G75900, AT2G23540, AT5G23370, ASN1, GPAT5, AT5G49350, GH9C1, AT1G80160, AT5G24080, AT4G27300, Hsp70b, AT5G24580, AT1G29000, AT1G71050, HHP1, AT1G32910, AT5G09520, LAC12, AT5G48480, AT1G54540, AT3G22800, AT5G13900, LACS4, TAA1, AT2G23790, SKS1, AT4G15700, MYB67, MYB93, MIOX2, NAC038, NAC058, NAC073, NAC075, AT1G09380, LP1, LTP4, NAT7, AT3G49190, OLEO3, AT4G35160, AT3G59850, PE11, AT5G19640, AT3G52790, AT1G68850, AT5G05340, AT5G14130, PAL2, AT3G09925, AT5G47635, IRX9, XTR6, AT3G22600, AT1G05450, BST1, CER3, EDA4, EXO, OXS3, AT5G09530, RPD1, TMN7, TBL19, TBL33, AT2G14830, AT5G23750, AT5G41400, ATL8, RIC4, AT2G33205, AT5G18840, SULTR1, AT5G11620, MYB84, UCC3, PGSIP3, AT5G27020, AT4G10265 and/or AT1G68360 gene in plants organisms and/or in plant tissues leads to the production of suberin in plants organisms and/or in plant tissues.

According to a preferred embodiment, said plant organisms are tobacco plant and/or apple plant, and/or wherein said plant tissues are leaves, fruits, roots and/or stems.

According to an example, the coding sequence of said orthologous gene of MYB93 transcription factor is the genes MDP0000320772 (SEQ-ID NO:1) and/or MDP0000228252 (SEQ-ID NO:2).

### Advantages of the invention

The invention is particularly interesting in that it provides a reliable source of suberin which then can be depolymerized and transformed in very useful material. The process of the present invention represents an important source of biopolymers that can be used in the industry.

### Description of an embodiment

The invention relates to the controlled production process of the suberin polymer (*i.e.* a copolyester) by the use of DNA sequence genes, named MDP0000320772 (SEQ-ID NO:1) isolated from *Malus x domestica* cv. Cox Orange pippin and/or orthologous genes of MYB93 transcription factor and coding a MYB-family transcription factor, named MYB93 and similar to AtMYB93 (*Arabidopsis thaliana* model plant), which is able to finely regulate the expression of the suberin down-stream synthesis dedicated enzymes, such as those described in table 1 below.

These suberin synthesis genes are found in plant organisms, for example tobacco plant (*Nicotiana benthamiana*) and/or apple plant, or plant tissues, for example plant leaves, fruits, roots and/or stems.

Suberin productive transformed plants, tissues or cells are obtained via the use of the bacterium *Agrobacterium tumefaciens* carrying a plasmid in which the DNA sequence genes coding for the MYB93 transcription factor is inserted. The plasmid, or the vector, comprises an inducible promotor or a constitutive promotor. An example of constitutive promotor is CaMV 35S. An example of inducible promotor is XVE, OLexA (induced by oestrogens), Alc, AlcA (induced by ethanol) or Gmhsp17.3 (induced by heat shock).

**Table 1: MYB93-like activated genes coded according to Arabidopsis thaliana orthologous genes (the corresponding gene code in Nicotiana benthamiana is also indicated).**

| **Gene code In *Nicotiana benthamiana*** | **Annotation description (*Arabidopsis thaliana* nr database)** | **Gene symbol (Arabidopsis thaliana nr database)** |
|---|---|---|
| Nbv5tr6228038 | 24-sterol c-methyltransferase | SMT1 |
| Nbv5tr6328789 | 3-ketoacyl- synthase 1 | KCS1 |
| Nbv5tr6222668 | 3-ketoacyl- synthase 4 | KCS4 |
| Nbv5tr6215803 | 4-coumarate-- ligase 1 | 4CL1 |
| Nbv5tr6243580 | 4-coumarate-- ligase 3 | 4CL3 |
| Nbv5tr6236478 | abc transporter g family member 1 | A T2G39350 |
| Nbv5tr6217020 | abc transporter g family member 11 | WBC11 |
| Nbv5tr6206538 | abc transporter g family member 16 | A T3G55090 |
| Nbv5tr6220564 | abc transporter g family member 2 | A T2G37360 |
| Nbv5tr6391581 | abc transporter g family member 23 | AT5G19410 |
| Nbv5tr6236569 | abc transporter g family member 32 | PDR4 |
| Nbv5tr6244229 | agamous-like mads-box protein agl21 | AGL21 |
| Nbv5tr6389537 | alpha beta fold family protein | AT4G24140 |
| Nbv5tr6314011 | alpha beta fold family protein | AT4G36610 |
| Nbv5tr6313510 | aluminum induced protein with ygl and Irdr motifs | AT4G27450 |
| Nbv5tr6235112 | annexin d8 | ANNAT8 |
| Nbv5tr6235088 | aspartyl protease family protein | AT2G03200 |
| Nbv5tr6220091 | at4g32480 f8b4_180 | AT 4G32480 |
| Nbv5tr6252744 | at-hook dna-binding family protein | AT4G17800 |
| Nbv5tr6268817 | bam3_arath ame: full=beta-amylase chloroplastic | CT-BMY |
| Nbv5tr6217187 | beta-tonoplast intrinsic protein | BETA-TIP |
| Nbv5tr6200335 | bifunctional inhibitor lipid-transfer protein seed storage 2s albumin superfamily protein | AT2G44300 |
| Nbv5tr6223644 | bifunctional inhibitor lipid-transfer protein seed storage 2s albumin-like protein | AT3G22620 |
| Nbv5tr6395628 | bifunctional sn-glycerol-3-phosphate 2-o-acyltransferase phosphatase | GPAT4 |
| Nbv5tr6318400 | bifunctional sn-glycerol-3-phosphate 2-o-acyltransferase phosphatase | GPAT6 |
| Nbv5tr6215068 | btb and taz domain protein 4 | BT4 |
| Nbv5tr6227571 | cellulose-synthase-like c12 | CSLC12 |
| Nbv5tr6237322 | class i glutamine amidotransferase-like protein | AT5G38200 |
| Nbv5tr6232726 | csplm_arath ame: full=casp-like protein 1d1 short= 1d1 | AT4G15610 |
| Nbv5tr6223406 | csplw_arath ame: full=casp-like protein 1b1 short= 1b1 | A T5G44550 |
| Nbv5tr6199034 | cupredoxin superfamily protein | AT5G07475 |
| Nbv5tr6220824 | cytochrome family subfamily polypeptide 1 | CYP94C1 |
| Nbv5tr6340302 | cytochrome family subfamily polypeptide 10 | CYP96A10 |
| Nbv5tr6346089 | cytochrome family subfamily polypeptide 8 | CYP86A8 |
| Nbv5tr6408715 | cytochrome p450 86a1 | CYP86A1 |
| Nbv5tr6237440 | cytochrome p450 86b1 | CYP86B1 |
| Nbv5tr6233583 | diaminopimelate decarboxylase 2 | A T5G11880 |
| Nbv5tr6203380 | diphenol oxidase | IRX12 |
| Nbv5tr6200724 | early nodulin-like protein 17 | ENODL17 |
| Nbv5tr6216023 | esterase lipase domain-containing protein | AT2G18360 |
| Nbv5tr6237694 | ethylene-responsive transcription factor wri1 | WRI1 |
| Nbv5tr6203422 | fatty acyl- reductase cer4 | CER4 |
| Nbv5tr6396810 | galactinol--sucrose galactosyltransferase 6 | DIN10 |
| Nbv5tr6222730 | gdsl esterase lipase | AT1G74460 |
| Nbv5tr6202105 | gdsl esterase lipase | AT5G03610 |
| Nbv5tr6216569 | gdsl esterase lipase | AT5G22810 |
| Nbv5tr6291143 | gdsl esterase lipase | AT5G37690 |
| Nbv5tr6207792 | gdsl esterase lipase exl3 | AT1G75900 |
| Nbv5tr6222428 | gdsl-motif lipase hydrolase | AT2G23540 |
| Nbv5tr6234393 | gem-like protein 8 | AT5G23370 |
| Nbv5tr6336275 | glutamine-dependent asparagine synthetase | ASN1 |
| Nbv5tr6232718 | glycerol-3-phosphate acyltransferase 5 | GPAT5 |
| Nbv5tr6232901 | glycine-rich protein | AT5G49350 |
| Nbv5tr6391909 | glycosyl hydrolase 9c1 | GH9C1 |
| Nbv5tr6236952 | glyoxylase i 7 | AT1G80160 |
| Nbv5tr6228455 | g-type lectin s-receptor-like serine threonine protein kinase | AT5G24080 |
| Nbv5tr6247978 | g-type lectin s-receptor-like serine threonine-protein kinase sd1-1 | AT 4G27300 |
| Nbv5tr6217644 | heat shock protein 70b | Hsp70b |
| Nbv5tr6225223 | heavy metal transport detoxification domain-containing protein | AT5G24580 |
| Nbv5tr6217989 | heavy-metal-associated domain-containing protein | AT1G29000 |
| Nbv5tr6199828 | heavy-metal-associated domain-containing protein | AT1G71050 |
| Nbv5tr6363705 | heptahelical transmembrane protein1 | HHP1 |
| Nbv5tr6203661 | hxxxd-type acyl-transferase-like protein | AT1G32910 |
| Nbv5tr6206592 | hydroxyproline-rich glycoprotein family protein | AT5G09520 |
| Nbv5tr6223276 | laccase 12 | LAC12 |
| Nbv5tr6199430 | lactoylglutathione lyase glyoxalase i-like protein | AT5G48480 |
| Nbv5tr6223877 | late embryogenesis abundant hydroxyproline-rich glycoprotein | AT1G54540 |
| Nbv5tr6235965 | leucine-rich repeat extensin-like protein 6 | A T3G22800 |
| Nbv5tr6199910 | lipid transfer-like protein vas | AT5G13900 |
| Nbv5tr6246568 | long chain acyl- synthetase 4 | LACS4 |
| Nbv5tr6204044 | l-tryptophan-pyruvate aminotransferase 1 | TAA1 |
| Nbv5tr6214962 | mcu2_arath ame: full=calcium uniporter protein mitochondrial flags: precursor | AT2G23790 |
| Nbv5tr6200242 | monocopper oxidase-like protein sks1 | SKS1 |
| Nbv5tr6224169 | monothiol glutaredoxin-s3 | AT4G15700 |
| Nbv5tr6217099 | myb domain protein 67 | MYB67 |
| Nbv5tr6229127 | myb domain protein 93 | MYB93 |
| Nbv5tr6235613 | myo-inositol oxygenase 2 | MIOX2 |
| Nbv5tr6226272 | nac domain containing protein 38 | NAC038 |
| Nbv5tr6224556 | nac domain containing protein 58 | NAC058 |
| Nbv5tr6205412 | nac domain containing protein 73 | NAC073 |
| Nbv5tr6222834 | nac domain containing protein 75 | NAC075 |
| Nbv5tr6221699 | nodulin 21-like transporter family protein | AT1G09380 |
| Nbv5tr6200284 | non-specific lipid-transfer protein 1 | LP1 |
| Nbv5tr6201754 | non-specific lipid-transfer protein 4 | LTP4 |
| Nbv5tr6290585 | nucleobase-ascorbate transporter 7 | NAT7 |
| Nbv5tr6314641 | o-acyltransferase (wsd1-like) family protein | AT3G49190 |
| Nbv5tr6307449 | oleo3_arath ame: full=oleosin kda | OLEO3 |
| Nbv5tr6214000 | o-methyltransferase-like protein | AT4G35160 |
| Nbv5tr6369799 | pectin lyase-like superfamily protein | AT3G59850 |
| Nbv5tr6216642 | pectinesterase 11 | PE11 |
| Nbv5tr6206160 | peptide nitrate transporter | AT5G19640 |
| Nbv5tr6217608 | peptidoglycan-binding domain-containing protein | AT3G52790 |
| Nbv5tr6227940 | peroxidase 11 | AT1G68850 |
| Nbv5tr6222399 | peroxidase 52 | AT5G05340 |
| Nbv5tr6235366 | peroxidase 55 | AT5G14130 |
| Nbv5tr6236806 | phenylalanine ammonia-lyase 2 | PAL2 |
| Nbv5tr6243328 | pollen ole e 1 allergen and extensin family protein | AT3G09925 |
| Nbv5tr6231268 | pollen ole e 1 allergen and extensin family protein | AT5G47635 |
| Nbv5tr6394602 | probable beta- -xylosyltransferase irx9 | IRX9 |
| Nbv5tr6200854 | probable xyloglucan endotransglucosylase hydrolase protein 23 | XTR6 |
| Nbv5tr6217812 | protease inhibitor seed storage lipid transfer protein family protein | AT3G22600 |
| Nbv5tr6235917 | protease inhibitor seed storage Itp family protein | AT1G05450 |
| Nbv5tr6203465 | protein deformed root hairs 4 | BST1 |
| Nbv5tr6246004 | protein eceriferum 3 | CER3 |
| Nbv5tr6207869 | protein embryo sac development arrest 4 | EDA4 |
| Nbv5tr6236780 | protein exordium | EXO |
| Nbv5tr6228779 | protein oxidative stress 3 | OXS3 |
| Nbv5tr6232591 | protein pro-glu-leu | A T5G09530 |
| Nbv5tr6212895 | protein root primordium defective 1 | RPD1 |
| Nbv5tr6215888 | protein transmembrane nine 7 | TMN7 |
| Nbv5tr6223226 | protein trichome birefringence-like 19 | TBL19 |
| Nbv5tr6202866 | protein trichome birefringence-like 33 | TBL33 |
| Nbv5tr6216437 | regulator of vps4 activity in the mvb pathway protein | AT2G14830 |
| Nbv5tr6203790 | remorin family protein | AT5G23750 |
| Nbv5tr6217669 | ring u-box superfamily protein | AT5G41400 |
| Nbv5tr6397663 | ring-h2 finger protein atl8 | ATL8 |
| Nbv5tr6237939 | rop-interactive crib motif-containing protein 4 | RIC4 |
| Nbv5tr6226886 | serinc-domain containing serine and sphingolipid biosynthesis protein | AT2G33205 |
| Nbv5tr6202392 | sugar transporter erd6-like 16 | AT5G18840 |
| Nbv5tr6268371 | sulfate transporter | SULTR1 |
| Nbv5tr6232134 | swim zinc finger family protein mitogen-activated protein kinase kinase kinase - related | AT5G11620 |
| Nbv5tr6218132 | transcription factor rax3 | MYB84 |
| Nbv5tr6232053 | uclacyanin 3 | UCC3 |
| Nbv5tr6223206 | udp-glucuronate:xylan alpha-glucuronosyltransferase 2 | PGSIP3 |
| Nbv5tr6232339 | weak similarity to elegans transposase (tr:g1125840) | AT5G27020 |
| Nbv5tr6264015 | wound-responsive protein | AT4G10265 |
| Nbv5tr6222913 | zinc finger-related protein | AT1G68360 |

The suberin production was assessed using delipidized extracts, which were then depolymerized using any type of with ester-breaking chemical reactions, such as:

Methanolysis with NaOMe catalyst (Graca J., Pereira H., Phytochem. Anal., 2000, 11, 45-51) can be employed.

Otherwise, alkaline hydrolysis with KOH in water/ethanol (Sousa A. F., et al., ChemSusChem, 2008, 1, 1020-1025) can be used.

Alternatively, a solution of trifluoroboron in methanol can be employed (Franke R. et al., Phytochem., 2005, 66, 2643-2658).

Alternatively yet, sulfuric acid in methanol can be used (Vishwanath S. J. et al., Plant Physiol., 2013, 163, 1118-1132).

Less aggressive techniques than alkaline hydrolysis like base and acid-catalyzed methanolysis, and hydrogenolysis, are preferred for analytical convenience (Kolattukudy, Advances in Biochemical Engineering/Biotechno., 2001, 71, 1-49).

The composition of the resulting depolymerized fraction is then analysed using a GC-MS analysis.

### Example 1 :

The stable expression of a MYB93-like transcription factor or an orthologous gene of MYB93 transcription factor in plant cells was also performed in accordance with the following experimental protocol:
**a.** The MDP0000320772 contig (SEQ-ID NO:1) is isolated from a cDNA library made from RNA extracted from *Malus x domestica* cv. "Cox Orange Pippin".
**b.** The following primers are used: Forward(F) 5'-CACCATGGGAAGATCTCCTT-3', Reverse(R) 5'-AGCAATCTCATTGGAAAAAGG-3' using the Q5 High Fidelity 2X Master Mix (New England Biolabs, Ipswich, MA, USA) following the manufacturer's instructions (Tm=60°C).
**c.** The PCR product is purified using the QIAquick PCR purification Kit (QIAGEN, Leusden, The Netherlands), cloned into the pGEM-T easy vector with a 3:1 (insert/vector ratio) and transformed into the *E. coli* JM109 competent cells following the manufacturer's guidelines (PROMEGA, Fitchburg, WI, USA).
**d.** Transformed cells are selected on Xgal/IPTG plate. Ten positive colonies are validated by PCR (Tm=55°C) using the M13 primers (F: 5'-CGCCAGGGTTTTCCCAGTCACGAC-3', R: 5'-TCACACAGGAAACAGCTATGAC-3'), sequence using a 3500 Genetic Analyzer using the BigDye terminator v3.1 kit (Thermo Fisher, Waltham, MA, USA) and blast against the *Malus x domestica* genome v1.
**e.** Plasmids are extracted using the QIAprep Spin Miniprep Kit (QIAGEN, Leusden, The Netherlands).
**f.** The MDP00003207772 fragment (SEQ-ID NO:1) was then inserted in the pENTR/D TOPO vector. The recombination steps are performed following the LR clonase cloning protocol (Invitrogen) using the pENTR/D TOPO::MDP0000320772 and PMDC7 vectors (Curtis and Grossniklauss, 2003).
**g.** Plant cells are cultured in Murashige and Skoog medium (MS medium). Cells in suspension are subcultured every seventh day (1 ml of cells into 30 ml of liquid media).
**h.** *Agrobacterium tumefaciens* GV3101-pMP90 strain (PMDC7::MDP0000320772) is grown in a 50 mL LB liquid medium supplemented with gentamycin (30 mg/L), rifampicin (10 mg/L), kanamycin (50 mg/L) and acetosyringone (30 mg/L).
**i.** The 100 mL baffled base Erlenmeyer flask is agitated at 130 rpm/28°C for 48 h. Culture is then centrifuged at 4000 rpm for 10 min, re-suspended into a 50 mM MES (pH 5.6), 10mM MgSO₄ infiltration buffer supplemented with 150 mg/L acetosyringone.
**j.** After a 2 hours of incubation, PMDC7::MDP0000320772 *A*. *tumefaciens* culture is adjusted at OD600=2
**k.** 5 mL plant cell culture were applied in a 100 mm Petri dish **I.** Add 5 µL acetosyringone to plant cell culture (stock is 150 mg/mL in DMSO), swirl gently to mix
**m.** Add the *Agrobacterium tumefaciens* strain to the plate. Use different amount of bacteria *i.e.* 25 µL and 100 µL
**n.** Cover plate with parafilm and incubate two days at 25°C
**o.** Transfer cells in 15 mL centrifuge tube
**p.** Let the cells settle in the centrifuge tube and remove the supernatant. Add 10ml MS medium to the cell and mix gently.
**q.** Repeat step **p** two more times using MS medium
**r.** Repeat step **p** with MS medium with 500 µg/mL carbenicilin
**s.** Put between 1-5 ml of washed cells onto a petri dish with MS supplemented with BASTA (20 µg/mL) and carbenicilin (500 µg/mL)
**t.** After 14 days BASTA under light exposure, resistant transformant calli should appear green whereas non transformant calli should appear bleached or yellow. Transfer micro-calli on a new selective plate (MS supplemented with BASTA (20 µg/mL) and carbenicilin (500 µg/mL))
**u.** Allow calli to grow for 7 to 10 days
**v.** Repeat steps **t-u** at least 3 times
**w.** Transfer one callus in a 1L baffled Flask filled with culture medium: 4.3 g Murashige and Skoog salt (M0221, Duchefa), 1mg thiamine hydrochloride, 100mg myo-inositol, 181 µL 2,4-Dichlorophenoxyacetic acid (5mM), 30g sucrose adjusted at pH=5.8. Shake vigorously for 2 min.
**x.** Incubate at 22°C for 10 days with 140 rpm agitation
**y.** Add 17-β-oestradiol at 10µM final
**z.** Incubate at 22°C for 10 days with 140 rpm agitation
**aa**.Centrifuge the culture at 13000g/10min
**bb**.Collect both pellet and supernatant
**cc.** Extract the lipid fraction and suberin fraction

### Example 2

The stable expression of a MYB93-like transcription factor or an orthologous gene of MYB93 transcription factor in plant cells was performed in accordance with the following experimental protocol:
**a.** The MDP0000320772 contig (SEQ-ID NO:1) is isolated from a cDNA library made from RNA extracted from *Malus x domestica* cv. "Cox Orange Pippin".
**b.** The following primers are used: Forward(F) 5'-CACCATGGGAAGATCTCCTT-3', Reverse(R) 5'-AGCAATCTCATTGGAAAAAGG-3' using the Q5 High Fidelity 2X Master Mix (New England Biolabs, Ipswich, MA, USA) following the manufacturer's instructions (Tm=60°C).
**c.** The PCR product is purified using the QIAquick PCR purification Kit (QIAGEN, Leusden, The Netherlands), cloned into the pGEM-T easy vector with a 3:1 (insert/vector ratio) and transformed into the *E. coli* JM109 competent cells following the manufacturer's guidelines (PROMEGA, Fitchburg, WI, USA).
**d.** Transformed cells are selected on Xgal/IPTG plate. Ten positive colonies are validated by PCR (Tm=55°C) using the M13 primers (F: 5'-CGCCAGGGTTTTCCCAGTCACGAC-3', R: 5'-TCACACAGGAAACAGCTATGAC-3'), sequence using a 3500 Genetic Analyzer using the BigDye terminator v3.1 kit (Thermo Fisher, Waltham, MA, USA) and blast against the *Malus x domestica* genome v1.
**e.** Plasmids are extracted using the QIAprep Spin Miniprep Kit (QIAGEN, Leusden, The Netherlands).
**f.** The following steps are performed following the LR clonase cloning protocol (Invitrogen) with the pENTR/D TOPO and pEarleyGate103 (p103) vectors (Earley *et al.* 2006).
**g.** Plant cells are cultured in Murashige and Skoog medium (MS medium). Cells in suspension are subcultured every seventh day (1 ml of cells into 30 ml of liquid media).
**h.** *Agrobacterium tumefaciens* GV3101-pMP90 strain (p103::MDP0000320772) is grown in a 50 mL LB liquid medium supplemented with gentamycin (30 mg/L), rifampicin (10 mg/L), kanamycin (50 mg/L) and acetosyringone (30 mg/L).
**i.** The 100 mL baffled base Erlenmeyer flask is agitated at 130 rpm/28°C for 48 h. Culture is then centrifuged at 4000 rpm for 10 min, re-suspended into a 50 mM MES (pH 5.6), 10mM MgSO₄ infiltration buffer supplemented with 150 mg/L acetosyringone.
**j.** After a 2 hours of incubation, p103::MDP0000320772 *A. tumefaciens* culture is adjusted at OD600=2
**k.** 5 mL plant cell culture were applied in a 100 mm Petri dish
**l.** Add 5 µL acetosyringone to plant cell culture (stock is 150 mg/mL in DMSO), swirl gently to mix
**m.** Add the *Agrobacterium tumefaciens* strain to the plate. Use different amount of bacteria *i.e.* 25 µL and 100 µL
**n**. Cover plate with parafilm and incubate two days at 25°C
**o.** Transfer cells in 15 mL centrifuge tube
**p.** Let the cells settle in the centrifuge tube and remove the supernatant
**q.** Add 10ml MS medium to the cell and mix gently.
**r.** Repeat steps **p-q** two more times using MS medium
**s.** Repeat steps **p-q** with MS medium with 500 µg/mL carbenicilin
**t.** Put between 1-5 ml of washed cells onto a petri dish with MS supplemented with BASTA (20 µg/mL) and carbenicilin (500 µg/mL)
**u.** After 14 days BASTA under light exposure, resistant transformant calli should appear green whereas non transformant calli should appear bleached or yellow
**v.** Transfer micro-calli on a new selective plate (MS supplemented with BASTA (20 µg/mL) and carbenicilin (500 µg/mL))
**w.** Allow calli to grow for 7 to 10 days
**x.** Repeat steps **v-w** at least 3 times

## Claims

1. Process for the production of a suberin polymer, comprising the following steps:
a) introducing by transformation a gene with a sequence corresponding to SEQ-ID NO:1 into plant cells from a plant organism and/or a plant tissue leading to genetically engineered plant cells;
b) stimulating said genetically engineered plant cells; and,
c) isolating a suberin polymer that is produced as result of step (b)
wherein said step (a) is performed by inserting at least one vector comprising a gene with a sequence corresponding to SEQ-ID NO:1 into a *Agrobacterium tumefaciens* and by infecting said plant cells from a plant organism and/or a plant tissue with said Agrobacterium tumefaciens, said at least one vector further comprising an inducible promotor.

2. Process according to claim 1, wherein said inducible promotor is induced by oestrogens, ethanol or by a heat shock.

3. Process according to any one of claims 1-2, wherein said step (b) is performed with hormones, preferably oestrogens.

4. Process for the production of monomers of suberin, comprising:
a) the process of the production of suberin in accordance with any one of claims 1 -3, and the subsequent steps of
b) removing the lipids contained in the suberin so as to produce a delipidized suberin; and
c) depolymerizing said delipidized suberin so as to produce the monomers of suberin.

5. Process according to claim 4, wherein said step (c) is performed by ester-breaking chemical reactions.

6. Process according to any one of claims 1 -5, wherein said plant organisms are tobacco plant and/or apple plant, and/or wherein said plant tissues are leaves, fruits, roots and/or stems.

7. Use of a gene with a sequence corresponding to SEQ-ID NO:1 as activator for regulating with an inducible promotor the expression of SMT1, KCS1, KCS4, 4CL1, 4CL3, AT2G39350, WBC11, AT3G55090, AT2G37360, AT5G19410, PDR4, AGL21, AT4G24140, AT4G36610, AT4G27450, ANNAT8, AT2G03200, AT4G32480, AT4G17800, CT-BMY, BETA-TIP, AT2G44300, AT3G22620, GPAT4, GPAT6, BT4, CSLC12, AT5G38200, AT4G15610, AT5G44550, AT5G07475, CYP94C1, CYP96A10, CYP86A8, CYP86A1, CYP86B1, AT5G11880, IRX12, ENODL17, AT2G18360, WRI1, CER4, DIN10, AT1G74460, AT5G03610, AT5G22810, AT5G37690, AT1G75900, AT2G23540, AT5G23370, ASN1, GPAT5, AT5G49350, GH9C1, AT1G80160, AT5G24080, AT4G27300, Hsp70b, AT5G24580, AT1G29000, AT1G71050, HHP1, AT1G32910, AT5G09520, LAC 12, AT5G48480, AT1G54540, AT3G22800, AT5G13900, LACS4, TAA1, AT2G23790, SKS1, AT4G15700, MYB67, MYB93, MIOX2, NAC038, NAC058, NAC073, NAC075, AT1G09380, LP1, LTP4, NAT7, AT3G49190, OLEO3, AT4G35160, AT3G59850, PE11, AT5G19640, AT3G52790, AT1G68850, AT5G05340, AT5G14130, PAL2, AT3G09925, AT5G47635, IRX9, XTR6, AT3G22600, AT1G05450, BST1, CER3, EDA4, EXO, OXS3, AT5G09530, RPD1, TMN7, TBL19, TBL33, AT2G14830, AT5G23750, AT5G41400, ATL8, RIC4, AT2G33205, AT5G18840, SULTR1, AT5G11620, MYB84, UCC3, PGSIP3, AT5G27020, AT4G10265 and/or AT1G68360 gene in plant organisms and/or in plant tissues.

8. Use according to claim 7, wherein the expression of SMT1, KCS1, KCS4, 4CL1, 4CL3, AT2G39350, WBC11, AT3G55090, AT2G37360, AT5G19410, PDR4, AGL21, AT4G24140, AT4G36610, AT4G27450, ANNAT8, AT2G03200, AT4G32480, AT4G 17800, CT-BMY, BETA-TIP, AT2G44300, AT3G22620, GPAT4, GPAT6, BT4, CSLC12, AT5G38200, AT4G15610, AT5G44550, AT5G07475, CYP94C1, CYP96A10, CYP86A8, CYP86A1, CYP86B1, AT5G1 1880, IRX12, ENODL17, AT2G18360, WRI 1, CER4, DIN10, AT1 G74460, AT5G03610, AT5G22810, AT5G37690, AT1 G75900, AT2G23540, AT5G23370, ASN1, GPAT5, AT5G49350, GH9C1, AT1 G80160, AT5G24080, AT4G27300, Hsp70b, AT5G24580, AT1 G29000, AT1 G71050, HHP1, AT1 G32910, AT5G09520, LAC12, AT5G48480, AT1 G54540, AT3G22800, AT5G13900, LACS4, TAA1, AT2G23790, SKS1, AT4G15700, MYB67, MYB93, MIOX2, NAC038, NAC058, NAC073, NAC075, AT1 G09380, LP1, LTP4, NAT7, AT3G49190, OLEO3, AT4G35160, AT3G59850, PE11, AT5G19640, AT3G52790, AT1 G68850, AT5G05340, AT5G14130, PAL2, AT3G09925, AT5G47635, IRX9, XTR6, AT3G22600, AT1 G05450, BST1, CER3, EDA4, EXO, OXS3, AT5G09530, RPD1, TMN7, TBL19, TBL33, AT2G14830, AT5G23750, AT5G41400, ATL8, RIC4, AT2G33205, AT5G18840, SULTR1, AT5G1 1620, MYB84, UCC3, PGSIP3, AT5G27020, AT4G10265 and/or AT1 G68360 gene in plants organisms and/or in plant tissues leads to the production of suberin in plants organisms and/or in plant tissues.

9. Use according to any one of claims 1-8, wherein said plant organisms are tobacco plant and/or apple plant, and/or wherein said plant tissues are leaves, fruits, roots and/or stems.

## Patentansprüche

1. Verfahren zur Herstellung eines Suberinpolymers umfassend die folgenden Schritte:
a) Einführen durch Transformation eines Genes mit einer Sequenz entsprechend SEQ-ID NO:1 in Pflanzenzellen von einem Pflanzenorganismus und/oder einem Pflanzengewebe, was zu gentechnisch veränderten Pflanzenzellen führt;
b) Stiumlieren der besagten gentechnisch veränderten Pflanzenzellen; und
c) Isolieren eines Suberinpolymers, das als Ergebnis von Schritt b) hergestellt ist,
wobei der besagte Schritt a) ausgeführt wird durch Einführen zumindest eines Vektors umfassend ein Gen mit einer Sequenz entsprechend SEQ-ID NO:1 in ein Agrobacterium tumefaciens und durch Infizieren der besagten Pflazenzellen von einem Pflanzenorganismus und/oder einem Pflanzengewebe mit dem besagten Agrobacterium tumefaciens, wobei der besagte zumindest eine Vektor weiter einen induzierbaren Promotor umfasst.

2. Verfahren gemäß Anspruch 1, wobei der besagte induzierbare Promotor induziert wird durch Östrogene, Ethanol oder durch einen Wärmeschock.

3. Verfahren gemäß einem der Ansprüche 1-2, wobei der besagte Schritt b) ausgeführt wird mit Hormonen, vorzugsweise Östrogenen.

4. Verfahren zur Herstellung von Monomeren von Suberin, umfassend:
a) das Verfahren der Herstellung von Suberin gemäß einem der Ansprüche 1-3 und die nachfolgenden Schritte des
b) Entfernens der Lipide, die in dem Suberin enthalten sind, um so ein delipidisiertes Suberin zu erzeugen; und
c) Depolymerisierens des besagten delipidisierten Suberins, um so die Monomere von Suberin herzustellen.

5. Verfahren gemäß Anspruch 4, wobei der besagte Schritt c) ausgeführt wird durch Ester-aufbrechende chemische Reaktionen.

6. Verfahren gemäß irgendeinem der Ansprüche 1-5, wobei die besagten Pflanzenorganismen eine Tabakpflanze und/oder eine Apfelpflanze sind und/oder wobei die besagten Pflanzengewebe Blätter, Früchte, Wurzeln und/oder Stängel sind.

7. Verwendung eines Gens mit einer Sequenz entsprechend SEQ-ID NO:1 als Aktivator zum Regulieren mit einem induzierbaren Promotor die Expression von SMT1, KCS1, KCS4, 4CL1, 4CL3, AT2G39350, WBC11, AT3G55090, AT2G37360, AT5G19410, PDR4, AGL21, AT4G24140, AT4G36610, AT4G27450, ANNAT8, AT2G03200, AT4G32480, AT4G17800, CT-BMY, BETA-TIP, AT2G44300, AT3G22620, GPAT4, GPAT6, BT4, CSLC12, AT5G38200, AT4G15610, AT5G44550, AT5G07475, CYP94C1, CYP96AIO, CYP86A8, CYP86A1, CYP86B1, AT5G11880, IRX12, ENODL17, AT2G18360, WRI1, CER4, DIN10, AT1G74460, AT5G03610, AT5G22810, AT5G37690, AT1G75900, AT2G23540, AT5G23370, ASN1, GPAT5, AT5G49350, GH9C1, AT1G80160, AT5G24080, AT4G27300, Hsp70b, AT5G24580, AT1G29000, AT1G71050, HHP1, AT1G32910, AT5G09520, LAC 12, AT5G48480, AT1G54540, AT3G22800, AT5G13900, LACS4, TAA1, AT2G23790, SKS1, AT4G15700, MYB67, MYB93, MIOX2, NAC038, NAC058, NAC073, NAC075, ATIG09380, LP1, LTP4, NAT7, AT3G49190, OLEO3, AT4G35160, AT3G59850, PE11, AT5G19640, AT3G52790, AT1G68850, AT5G05340, AT5G14130, PAL2, AT3G09925, AT5G47635, IRX9, XTR6, AT3G22600, AT1G05450, BST1, CER3, EDA4, EXO, OXS3, AT5G09530, RPD1, TMN7, TBL19, TBL33, AT2G14830, AT5G23750, AT5G41400, ATL8, RIC4, AT2G33205, AT5G18840, SULTR1, AT5G11620, MYB84, UCC3, PGSIP3, AT5G27020, AT4G10265 und/oder AT1G68360 Gen in Pflanzenorganismen und/oder Pflanzengeweben.

8. Verwendung gemäß Anspruch 7, wobei die Expression von SMT1, KCS1, KCS4, 4CL1, 4CL3, AT2G39350, WBC11, AT3G55090, AT2G37360, AT5G19410, PDR4, AGL21, AT4G24140, AT4G36610, AT4G27450, ANNAT8, AT2G03200, AT4G32480, AT4G17800, CT-BMY, BETA-TIP, AT2G44300, AT3G22620, GPAT4, GPAT6, BT4, CSLC12, AT5G38200, AT4G15610, AT5G44550, AT5G07475, CYP94C1, CYP96AIO, CYP86A8, CYP86A1, CYP86B1, AT5G11880, IRX12, ENODL17, AT2G18360, WRI1, CER4, DIN10, AT1G74460, AT5G03610, AT5G22810, AT5G37690, AT1G75900, AT2G23540, AT5G23370, ASN1, GPAT5, AT5G49350, GH9C1, AT1G80160, AT5G24080, AT4G27300, Hsp70b, AT5G24580, AT1G29000, AT1G71050, HHP1, AT1G32910, AT5G09520, LAC 12, AT5G48480, AT1G54540, AT3G22800, AT5G13900, LACS4, TAA1, AT2G23790, SKS1, AT4G15700, MYB67, MYB93, MIOX2, NAC038, NAC058, NAC073, NAC075, ATIG09380, LP1, LTP4, NAT7, AT3G49190, OLEO3, AT4G35160, AT3G59850, PE11, AT5G19640, AT3G52790, AT1 G68850, AT5G05340, AT5G14130, PAL2, AT3G09925, AT5G47635, IRX9, XTR6, AT3G22600, AT1 G05450, BST1, CER3, EDA4, EXO, OXS3, AT5G09530, RPD1, TMN7, TBL19, TBL33, AT2G14830, AT5G23750, AT5G41400, ATL8, RIC4, AT2G33205, AT5G18840, SULTR1, AT5G11620, MYB84, UCC3, PGSIP3, AT5G27020, AT4G10265 und/oder AT1 G68360 Gen in Pflanzenorganismen und/oder Pflanzengeweben zu der Herstellung von Suberin in Pflanzenorganismen und/oder Pflanzengeweben führt.

9. Verwendung gemäß irgendeinem der Ansprüche 1-8, wobei die besagten Pflanzenorganismen eine Tabakpflanze und/oder eine Apfelpflanze sind und/oder wobei die besagten Pflanzengewebe Blätter, Früchte, Wurzeln und/oder Stängel sind.

## Revendications

1. Procédé pour la production d'un polymère de subérine comprenant les étapes suivantes dans lesquelles :
a) on introduit, par l'intermédiaire d'une transformation, un gène comprenant une séquence correspondant à la SEQ ID NO:1 dans des cellules végétales à partir d'un organisme végétal et/ou d'un tissu végétal, ce qui donne lieu à des cellules végétales génétiquement modifiées ;
b) on stimule lesdites cellules végétales génétiquement modifiées ; et
c) on isole un polymère de subérine que l'on obtient à titre de résultat de l'étape (b) ;
dans lequel, ladite étape (a) est mise en oeuvre en insérant au moins un vecteur comprenant un gène muni d'une séquence correspondant à la SEQ ID NO:1 dans une bactérie *Agrobacterium tumefactiens* et en infectant lesdites cellules végétales à partir d'un organisme végétal et/ou d'un tissu végétal avec ladite bactérie Agrobacterium tumefactiens, ledit au moins un vecteur comprenant en outre un promoteur inductible.

2. Procédé selon la revendication 1, dans lequel ledit promoteur inductible est induit par des oestrogènes, par l'intermédiaire d'éthanol ou via un choc thermique.

3. Procédé selon l'une quelconque des revendications 1 à 2, dans lequel ladite étape (b) est mise en oeuvre avec des hormones, de préférence avec des oestrogènes.

4. Procédé pour la production de monomères de subérine, comprenant :
a) le procédé de production de subérine selon l'une quelconque des revendications 1 à 3 ;
ainsi que les étapes ultérieures consistant à :
b) éliminer les lipides que contient la subérine de façon à obtenir une subérine délipidée ; et
c) dépolymériser ladite subérine délipidée de façon à obtenir les monomères de subérine.

5. Procédé selon la revendication 4, dans lequel ladite étape (c) est mise en oeuvre via des réactions chimiques de rupture d'esters.

6. Procédé selon l'une quelconque des revendications 1 à 5, dans lequel lesdits organismes végétaux représentent une plantation de tabac et/ou une plantation de pommiers et/ou dans lequel lesdits tissus végétaux représentent des feuilles, des fruits, des racines et/ou des tiges.

7. Utilisation d'un gène comprenant une séquence correspondant à la SEQ ID NO:1 à titre d'activateur pour une régulation, avec un promoteur inductible, de l'expression des gènes SMT1, KCS1, KCS4, 4CL1, 4CL3, AT2G39350, WBC11, AT3G55090, AT2G37360, AT5G19410, PDR4, AGL21, AT4G24140, AT4G36610, AT4G27450, ANNAT8, AT2G03200, AT4G32480, AT4G17800, CT-BMY, BETA-TIP, AT2G44300, AT3G22620, GPAT4, GPAT6, BT4, CSLC12, AT5G38200, AT4G15610, AT5G44550, AT5G07475, CYP94C1, CYP96A10, CYP86A8, CYP86A1, CYP86B1, AT5G11880, IRX12, ENODL17, AT2G18360, WRI1, CER4, DIN10, AT1G74460, AT5G03610, AT5G22810, AT5G37690, AT1G75900, AT2G23540, AT5G23370, ASN1, GPAT5, AT5G49350, GH9C1, AT1G80160, AT5G24080, AT4G27300, Hsp70b, AT5G24580, AT1G29000, AT1G71050, HHP1, AT1G32910, AT5G09520, LAC12, AT5G48480, AT1G54540, AT3G22800, AT5G13900, LACS4, TAA1, AT2G23790, SKS1, AT4G15700, MYB67, MYB93, MIOX2, NAC038, NAC058, NAC073, NAC075, AT1G09380, LP1, LTP4, NAT7, AT3G49190, OLEO3, AT4G35160, AT3G59850, PE11, AT5G19640, AT3G52790, AT1G68850, AT5G05340, AT5G14130, PAL2, AT3G09925, AT5G47635, IRX9, XTR6, AT3G22600, AT1G05450, BST1, CER3, EDA4, EXO, OXS3, AT5G09530, RPD1, TMN7, TBL19, TBL33, AT2G14830, ATSG23750, ATSG41400, ATL8, RIC4, AT2G33205, ATSG18840, SULTR1, ATSG11620, MYB84, UCC3, PGSIP3, ATSG27020, AT4G10265 et/ou AT1G68360 dans des organismes végétaux et/ou dans des tissus végétaux.

8. Utilisation selon la revendication 7, dans laquelle l'expression du gène SMT1, KCS1, KCS4, 4CL1, 4CL3, AT2G39350, WBC11, AT3G55090, AT2G37360, AT5G19410, PDR4, AGL21, AT4G24140, AT4G36610, AT4G27450, ANNAT8, AT2G03200, AT4G32480, AT4G17800, CT-BMY, BETA-TIP, AT2G44300, AT3G22620, GPAT4, GPAT6, BT4, CSLC12, AT5G38200, AT4G15610, AT5G44550, AT5G07475, CYP94C1, CYP96A10, CYP86A8, CYP86A1, CYP86B1, AT5G11880, IRX12, ENODL17, AT2G18360, WRI1, CER4, DIN10, AT1G74460, AT5G03610, AT5G22810, AT5G37690, AT1G75900, AT2G23540, AT5G23370, ASN1, GPAT5, AT5G49350, GH9C1, AT1G80160, AT5G24080, AT4G27300, Hsp70b, AT5G24580, AT1G29000, AT1G71050, HHP1, AT1G32910, AT5G09520, LAC12, AT5G48480, AT1G54540, AT3G22800, AT5G13900, LACS4, TAA1, AT2G23790, SKS1, AT4G15700, MYB67, MYB93, MIOX2, NAC038, NAC058, NAC073, NAC075, AT1G09380, LP1, LTP4, NAT7, AT3G49190, OLEO3, AT4G35160, AT3G59850, PE11, AT5G19640, AT3G52790, AT1G68850, AT5G05340, AT5G14130, PAL2, AT3G09925, AT5G47635, IRX9, XTR6, AT3G22600, AT1G05450, BST1, CER3, EDA4, EXO, OXS3, AT5G09530, RPD1, TMN7, TBL19, TBL33, AT2G14830, ATSG23750, ATSG41400, ATL8, RIC4, AT2G33205, ATSG18840, SULTR1, ATSG11620, MYB84, UCC3, PGSIP3, ATSG27020, AT4G10265 et/ou AT1G68360 dans des organismes végétaux et/ou dans des tissus végétaux donne lieu à la production de subérine dans des organismes végétaux et/ou dans des tissus végétaux.

9. Utilisation selon l'une quelconque des revendications 1 à 8, dans laquelle lesdits organismes végétaux représentent une plantation de tabac et/ou une plantation de pommiers et/ou dans lequel lesdits tissus végétaux représentent des feuilles, des fruits, des racines et/ou des tiges.


### SEQUENCE LISTING

<110> LIST
<120> Production of suberin
<140> EPLU93026
   <141> 2016-04-14
<160> 2
<170> BiSSAP 1.3.6
<210> 1
   <211> 1038
   <212> DNA
   <213> Malus domestica
<223> MDP0000320772
<400> 1
   atgggaagat ctccttgttg tgatgagagt ggccttaaga aaggcccttg gactcctgaa      60
   gaagatcaaa aacttatgaa atacattcag aaaaatggcc atggaagctg gagagccctt     120
   ccaaaacttg cagggttgaa cagatgcggc aagagttgca ggttaagatg gacaaactat     180
   cttaggccgg atataaagag agggaagttt tctcaagaag aagaacaaac aattcttaat     240
   ctccattcca tcctcggcaa caaatggtca gcaattgcaa gccacctacc cggtcggacg     300
   gacaatgaaa taaaaaattt ctggaacacc catttgaaga aaaagctaat tcagatgggt     360
   tttgatccga tgactcaccg gccccggact gatatcttct caagcttgcc tcatcttcta     420
   gctcttgtca atttgaaaga gcttctagac catggaagca gtcaccaatc cagttgggat     480
   gaacaagctt tgagattaca agcagaagct gctcaaatgg ctaggcttca atacctacaa     540
   tgtctcctcc aacctaataa caattccttt ggagctgcta ccccaaacaa cctaaatgac     600
   ttractgaca tggagaccat taatcttttg aattcactct gttcatctca gctggatatt     660
   caaaacccta ctgcacaata ccctgttggt agttttcaac cagtagtcca agataattca     720
   gtccctttct ctcatttgcc tgacttacaa gcctatccaa caccaccaag caaggacatg     780
   gttgatgatc aagttccaga tcaatttact gcagttttga gccaagatga aagctctcca     840
   aataatccat ggaatattcc ttcatcttca aataccactc catctcctac ttcggtggct     900
   cctcccgtga cagtgacaga gacttcagta agcaacaaca tgggtgatgc ttgcagcact     960
   tcaagctatg gaggagtggc tacttcagcg tggtctgacc ttctttttga ggaccctttt    1020
   tccaatgaga ttgcttaa                                                  1038
<210> 2
   <211> 1026
   <212> DNA
   <213> Malus domestica
<400> 2
   atgggaagat ctccttgttg tgatgagagt ggcctcaaga aaggcccttg gactcctgaa      60
   gaagatcaga aacttatgaa atacattcag aaaaatggcc atggaagctg gagagccctt     120
   ccaaaacttg cagggttgaa tagatgcggc aagagttgca ggttaagatg gacaaactat     180
   cttaggccgg atataaagag agggaagttt tctcaagaag aagaacaaac aattctgaat     240
   ctccattcca ttctcggcaa caaatggtca gcaattgcag gccacttacc cggtcggacg     300
   gacaatgaaa tcaaaaattt ctggaatacc catttgaaga aaaagctgat tcagatgggt     360
   tttgatccga tgactcaccg gccccggact gatatcttct ctagcttgcc tcatcttcta     420
   gctcttgtca atttgaaaga gcttttagac catggaagca gtcaccagtc ctgctgggat     480
   gaacaagctt tgagattaca agcagaagct tctcaaatgg ctkggcttca atatctacaa     540
   tgtctcctcc aycctaataa caattccttt ggagctgcta csccaaacaa cctaaatgar     600
   ttcattgaca tggagacatt taatcttttg aatwcactcg gttcatctca gctggatatt     660
   caagacccta ctgcacaata ccctgttggt gattttcaac cagtagtcca agataattca     720
   gtccctttct ctcatttgcc tgacttgcaa acatacccaa caccaccaaa caaggacatg     780
   gttaatgatc aagctccaga tcaatttact gctttgagcc aaggtgaaaa ctctccaaat     840
   aatccatgga atattccttc gtcttcaaat actactctat ctcctacttc tgtggctcct     900
   cccatgacag tgacagagac ttcagtaagc aacaacttgg gtgatgcttg cagcacttca     960
   agctatgcag gagtggctac ttcagcttgg tctgaccttc tttttgagga cccctttttt    1020
   cagtga                                                               1026